# EUROPÄISCHE PATENTSCHRIFT

(11) **EP 3 829 495 B1**
(45) Veröffentlichungstag und Bekanntmachung des Hinweises auf die Patenterteilung: **01.12.2021**
(21) Anmeldenummer: 19739564.3
(22) Anmeldetag: 10.07.2019
(51) Int. Cl.: A61F 2/64, A61F 2/68

(54) **BEFESTIGUNGSEINRICHTUNG ZUR BEFESTIGUNG EINES PROTHESENSCHAFTES AN EINEM PROTHESENKNIEGELENK UND PROTHESENKNIEGELENK**
FASTENING DEVICE FOR FASTENING A PROTHESIS SHAFT TO A PROSTHETIC KNEE JOINT, AND PROSTHETIC KNEE JOINT
DISPOSITIF DE FIXATION POUR LA FIXATION D'UNE TIGE DE PROTHÈSE À UNE ARTICULATION DE GENOU DE PROTHÈSE ET ARTICULATION DE GENOU DE PROTHÈSE

(30) Priorität: 30.07.2018 DE 102018118363
(43) Veröffentlichungstag der Anmeldung: 09.06.2021
(73) Patentinhaber: Ottobock SE & Co. KGaA, 37115 Duderstadt (DE)
(72) Erfinder: SCHOLL, Patrick, 04081-001 Indianópolis, São Paulo (BR)
(74) Vertreter: Gramm, Lins & Partner Patent- und Rechtsanwälte PartGmbB
(86) Internationale Anmeldenummer: PCT/EP2019/068533
(87) Internationale Veröffentlichungsnummer: WO 2020/025274

(56) Entgegenhaltungen:
- DE-A1-102010 031 723
- GB-A- 2 134 392
- US-A1- 2016 015 531

## Beschreibung

Die Erfindung betrifft eine Befestigungseinrichtung zur Befestigung eines Prothesenschaftes an einem Prothesenkniegelenk sowie ein Prothesenkniegelenk mit einer solchen Befestigungseinrichtung, wie in den Ansprüchen definiert.

Das Prothesenkniegelenk weist ein proximales Oberteil und ein distales Unterteil auf, die schwenkbar um eine Prothesenkniegelenksachse aneinander gelagert sind.

Prothetische Versorgungen an unteren Extremitäten sind notwendig, um bei fehlenden Gliedmaßen eine Annäherung an das natürliche Erscheinungsbild zu erreichen und nach Möglichkeit die Funktion einer natürlichen Gliedmaße nachzuahmen und zu ersetzen. Patienten mit einem fehlenden Kniegelenk benötigen ein Prothesenkniegelenk, das in der Regel an einem Prothesenschaft befestigt ist, der an einem Oberschenkelstumpf festgelegt wird. Zur Festlegung des Oberschenkelschaftes an dem Stumpf sind mehrere Systeme bekannt. Den Systemen gemeinsam ist, dass in dem Prothesenschaft der Oberschenkelstumpf gehalten ist und über ein Prothesenkniegelenk mit einem Unterschenkelrohr und einem Prothesenfuß die Verbindung zu dem Boden hergestellt wird. Das Prothesenkniegelenk ermöglicht eine schwenkbare Verbindung des Unterschenkelteils gegenüber dem Oberschenkelstumpf. Um ein möglichst natürliches Gangbild und ein möglichst natürliches äußeres Erscheinungsbild zu erreichen, wird die Prothesenkniegelenksachse nach Möglichkeit auf Höhe der natürlichen Kniegelenksachse des unversorgten, gesunden Beines angeordnet. Anderenfalls würde sich ein sehr ungleichmäßiges Gangbild ergeben. Ist der Oberschenkelstumpf vergleichsweise kurz, kann der Abstand über ein Oberschenkelrohr ausgeglichen werden.

Die DE 10 2009 051 668 A1 betrifft ein Kniegelenk für eine Prothese, umfassend eine Schwungphasensteuerung mit einem mit einem Oberschenkelverbindungsstück gekoppelten, gegen einen Zylinder bewegbaren Kolben, wobei der Zylinder mit einem Unterschenkelverbindungstück gekoppelt und gegen das Unterschenkelverbindungsstück um wenigstens eine Achse verschwenkbar ist. Der Zylinder ist über einen achsfernen Anlenkpunkt mit einem Federmittel verbunden, das sich an dem Unterschenkelverbindungsstück abstützt.

Die DE 10 2009 053 128 B4 betrifft ein Prothesenkniegelenk mit einem Pyramidenadapter zur Befestigung eines Prothesenschaftes. Distal zu dem Pyramidenadapter befindet sich ein Querlenker, in dem zwei Querbohrungen angeordnet sind. Die erste Querbohrung nimmt zwei vordere Verbindungshebel auf, die zweite Querbohrung einen Verbindungshebel. Der Verbindungshebel ist gelenkig an einer sogenannten Hemmung gelagert, die wiederum gelenkig an einem distalen Unterteil gelagert ist. Dazu beanstandet sind die beiden Verbindungshebel an dem Unterteil schwenkbar gelagert. Die Hemmung ist in einem Langloch in dem Unterteil gegen ein Federelement gelagert.

Die WO 03/092545 A2 betrifft ein Prothesenkniegelenk mit vier Achsbolzen, wobei die vier Achsbolzen einen Kniekopf, zwei Verbindungsplatten, eine Gabel und einen Sitz zur Verbindung mit einem Unterschenkelteil miteinander verbinden und eine dreieckige Stützung aufbauen, wobei der Angriffspunkt auf der Spitze des umgekehrten Dreiecks liegt, das von den vier Achsbolzen gebildet ist.

Die GB 2 134 392 A betrifft ein Prothesenkniegelenk mit einem Oberteil und einem Unterteil, die über vordere und hintere Lenker gelenkig miteinander verbunden sind. Bei der Beugebewegung des Prothesenkniegelenks wird Energie in einem Federelement gespeichert, sodass beim Beginn der Schwungphase die Extensionsbewegung unterstützt wird.

Die US 2016/0015531 A1 betrifft ein Prothesenkniegelenk in Vierlenkerbauweise. Die Beugebewegung des Prothesenkniegelenks wird beschränkt, indem die Rotation des Prothesenkniegelenks um die Rotationsachse durch Kompression von Dämpfungselementen und entsprechender Blockierung von Wellenlagern gebremst wird.

Die DE 10 2010 031 723 A1 betrifft ein Prothesenkniegelenk mit einem Oberteil, welches obere Anschlussmittel aufweise, und einem schwenkbar über vordere und hintere Lenker an dem Oberteil gelagerten Unterteil. Die Relativbewegung des Oberteils zum Unterteil treibt eine Vakuumpumpe an, die mit einem Eingang und einem Ausgang dem Prothesenkniegelenk zugeordnet ist.

Eine besondere Problematik ergibt sich bei Knieexartikulationspatienten. Bei einer Knieexartikulation bleibt der Oberschenkelknochen im Wesentlichen unverletzt, die distalen Kondylen des Oberschenkelknochens bleiben im Wesentlichen erhalten. Eine solche Versorgung ist für einen Patienten im Hinblick auf die Druckbelastung des Weichteilgewebes im Stumpfendbereich vorteilhaft. Problematisch bei Knieexartikulationen ist, dass ein Prothesenkniegelenk an das distale Ende des Prothesenschaftes angebracht werden muss, so dass sich eine zusätzliche Oberschenkelverlängerung beispielsweise durch einen Prothesenliner, den Prothesenschaft, ein Befestigungselement an dem Prothesenschaft zur Festlegung an dem Oberteil des Prothesenkniegelenkes und das Oberteil selbst ergibt. Der Abstand von dem Hüftgelenk zu der Prothesenkniegelenksachse ist mitunter signifikant größer als der Abstand von dem Hüftgelenk zur natürlichen Kniegelenksachse. Dies hat Nachteile sowohl beim Gehverhalten als auch beim Sitzen zur Folge, beispielsweise in öffentlichen Verkehrsmitteln mit eingeschränkter Beinfreiheit.

Aufgabe der vorliegenden Erfindung ist es daher, eine Befestigungseinrichtung und ein Prothesenkniegelenk bereitzustellen, mit denen diese Nachteile verringert oder vermieden werden können.

Erfindungsgemäß wird diese Aufgabe durch eine Befestigungseinrichtung mit den Merkmalen des Hauptanspruches sowie durch ein Prothesenkniegelenk mit den Merkmalen des nebengeordneten Anspruchs gelöst. Vorteilhafte Ausgestaltungen und Weiterbildungen der Erfindung sind in den Unteransprüchen, der Beschreibung sowie den Figuren offenbart.

Die Befestigungseinrichtung zur Befestigung eines Prothesenschaftes an einem Prothesenkniegelenk, das ein proximales Oberteil und ein distales Unterteil aufweist, die schwenkbar um eine Prothesenkniegelenksachse aneinander gelagert sind, weist eine distale Halterung zur Festlegung an dem Unterteil oder einem Unterschenkelrohr, das mit dem Unterteil verbunden ist, auf. Von der Halterung erstreckt sich zumindest eine Strebe in Proximalrichtung über die Prothesenkniegelenksachse hinaus. An der Strebe ist proximal zu der Prothesenkniegelenksachse eine Aufnahme zur schwenkbaren Lagerung des Prothesenschaftes um eine Schwenkachse angeordnet. Versetzt zu der Schaftschwenkachse, also exzentrisch zu der Schaftschwenkachse, ist zumindest ein Kraftübertragungselement an einem proximalen Lagerpunkt befestigt, um eine Schwenkbewegung des Prothesenschaftes zu übertragen. Das Kraftübertragungselement ist an einem distalen Lagerpunkt mit dem Oberteil dergestalt gekoppelt, dass eine Schwenkbewegung des Prothesenschaftes um die Schaftschwenkachse eine Schwenkbewegung des Oberteils des Prothesenkniegelenkes um die Prothesenkniegelenksachse bewirkt. Über die Befestigungseinrichtung ist es möglich, ein herkömmliches Prothesenkniegelenk auch für Patienten mit einer Knieexartikulation oder mit besonders langen Oberschenkelstümpfen zu verwenden. Das Prothesenkniegelenk kann als einfaches Sperrkniegelenk mit einer monozentrischen Schwenkachse, als ein Mehrlenkerkniegelenk, als Prothesenkniegelenk mit einer Dämpfereinrichtung und insbesondere als ein Prothesenkniegelenk mit einer elektronischen Steuerung zur Veränderung eines Extensions- und/oder Flexionswiderstandes ausgebildet sein. Konstruktive Änderungen an den Kniegelenken sind durch die Kopplung des Prothesenschaftes mit dem Prothesenkniegelenk über die Befestigungseinrichtung nicht notwendig. Trotz Vorhandensein eines sehr langen Oberschenkelstumpfes mit einem zusätzlichen Prothesenschaft, der die effektive Länge des Stumpfes noch vergrößert, ist es möglich, eine prothetische Versorgung mit Segmentlängen, die zu den Segmentlängen eines gesunden Beines korrespondieren, bereitzustellen. Dadurch wird ein verbessertes Gangverhalten sowie eine Minimierung der Oberschenkelverlängerung beim Sitzen erreicht. Die Strebe, die an dem Unterteil des Prothesenkniegelenkes oder auch an einem Unterschenkelrohr festgelegt und fixiert ist, ist starr mit dem Unterteil oder dem Unterschenkelrohr verbunden und steht in proximaler Richtung über die Prothesenkniegelenksachse hinaus. Die Aufnahme zur schwenkbaren Lagerung des Prothesenschaftes an der Strebe kann integral mit der Strebe ausgebildet sein, alternativ kann ein Bauteil oder ein Adapter an der Strebe befestigt sein, an dem der Prothesenschaft direkt oder indirekt festgelegt wird. Der Prothesenschaft kann unmittelbar an der Strebe oder der Aufnahme fixiert sein, dazu sind an dem Prothesenschaft entsprechende Aufnahmen, Zapfen oder Lager angeordnet oder ausgebildet, alternativ kann an dem Prothesenschaft ein Zwischenstück angeordnet sein, über das der Prothesenschaft mit der Strebe oder der Aufnahme gekoppelt ist. Eine direkte Anbindung des Prothesenschaftes an der Strebe oder an den Streben verringert die Anzahl der zu fertigenden Komponenten und damit auch das Gewicht. Werden Zwischenstücke, Bauteile oder Adapter vorgesehen, die zwischen der Strebe und dem Prothesenschaft angeordnet werden können, kann eine erleichterte Anpassung eines individuellen Prothesenschaftes an standardisierte Prothesenkomponenten, insbesondere an eine standardisierte Befestigungseinrichtung erfolgen.

Durch die exzentrisch oder versetzt zu der Schaftschwenkachse befestigte Kraftübertragungseinrichtung ist es möglich, Kräfte oder Momente, die durch eine Verschwenkung des Prothesenschaftes um die Schaftschwenkachse aufgebracht werden, in distale Richtung weiterzuleiten, so dass das Oberteil relativ zu dem Unterteil korrespondierend zu einer Verschwenkung des Prothesenschaftes relativ zu der Strebe verschwenkt wird. Das Kraftübertragungselement weist einen proximalen Lagerpunkt und einen distalen Lagerpunkt auf, wobei der distale Lagerpunkt mit dem Oberteil gekoppelt ist. Der distale Lagerpunkt kann entweder direkt an dem Oberteil ausgebildet sein oder über ein weiteres Bauteil mit dem Oberteil gekoppelt werden. Wird ein Bauteil zwischen dem Oberteil des Prothesenkniegelenkes und dem Kraftübertragungselement vorgesehen, ist eine erleichterte Anpassung an die individuellen Bedürfnisse möglich.

Der proximale Lagerpunkt kann an dem Prothesenschaft selbst oder an einem Hebel angeordnet sein, der um die Schaftschwenkachse schwenkbar und mit dem Prothesenschaft gekoppelt ist. Wird der proximale Lagerpunkt der Kraftübertragungseinrichtung an dem Prothesenschaft selbst ausgebildet, bildet der Prothesenschaft einen Teil des Mehrlenkergetriebes, das durch die zumindest eine Strebe, dem Prothesenschaft und die Kraftübertragungseinrichtung ausgebildet ist. Das distale Ende der Kraftübertragungseinrichtung kann ebenfalls schwenkbar mit dem Oberteil des Prothesenkniegelenkes gekoppelt sein, so dass drei schwenkbar aneinander gelagerte Hebel vorhanden sind, die wiederum durch das Prothesenkniegelenk miteinander gekoppelt sind. Alternativ zu einer Anordnung des proximalen Lagerpunktes unmittelbar an dem Prothesenschaft ist es möglich, dass ein Hebel ausgebildet ist, der drehfest und momentenübertragend mit dem Prothesenschaft gekoppelt ist, so dass bei einer Verschwenkung des Prothesenschaftes um die Schaftschwenkachse der Hebel mit verschwenkt wird. Durch die Ausgestaltung eines Hebels ist es möglich, die Befestigungseinrichtung als ein vorfertigbares Modul auszubilden, das an zwei Stellen mit dem vorhandenen Prothesenkniegelenk und an einer Stelle mit dem Prothesenschaft gekoppelt werden muss. Die Strebe ist an dem Unterteil festgelegt oder starr zu dem Unterteil an einer distal zu der Prothesenkniegelenksachse angeordneten Prothesenkomponente fixiert. Das Kraftübertragungselement ist distal mit dem Oberteil des Prothesenkniegelenkes gekoppelt und der Prothesenschaft ist dergestalt mit der Kraftübertragungseinrichtung schwenkbar an der zumindest einen Strebe gekoppelt, dass eine Verschwenkung um die Schaftschwenkachse eine Verlagerung des Kraftübertragungselementes bewirkt. Dies erleichtert die Zusammenstellung der endgültigen Prothese, insbesondere den Austausch von Prothesenkniegelenken mit standardisierten Aufnahmeeinrichtungen für die Befestigungseinrichtung. An der Befestigungseinrichtung selbst kann eine standardisierte Aufnahmeeinrichtung zur Festlegung des Prothesenschaftes angeordnet oder ausgebildet sein.

An der zumindest einen Strebe kann ein Bügel mit einer Aufnahmeeinrichtung zur Festlegung des Prothesenschaftes an dem Bügel gelagert sein. Die Aufnahmeeinrichtung kann als Teil eines Pyramidenadapters ausgebildet sein, der Bügel kann als einarmiger oder zweiarmiger Bügel ausgebildet sein. Aus Gründen der Stabilität sind bevorzugt zwei Bügel, ein medialer und ein lateraler Bügel vorgesehen, zwischen denen der Prothesenschaft aufgenommen und gelagert wird. Erfolgt eine Lagerung nicht unmittelbar an dem Prothesenschaft, kann zum Ausgleich von Maßabweichungen oder zur Anpassung an unterschiedliche Prothesenschaftgrößen eine Befestigung über einen Bügel erfolgen, der schwenkbar in der Aufnahme an der Strebe gelagert ist. Bei zwei Streben bietet sich ein zweiarmiger Bügel an, so dass eine mediale und laterale Lagerung und eine Verschwenkung um eine gemeinsame Schaftschwenkachse ermöglicht werden. An dem Bügel, beispielsweise zentral zwischen den Bügelenden, kann eine Aufnahmeeinrichtung, beispielsweise eine herkömmliche Pyramidenadapterkomponente angeordnet sein, so dass ein herkömmlicher Prothesenschaft mit den üblichen distalen Anschlussmitteln zur Festlegung an einem Prothesenkniegelenk verwendet werden kann, um eine Kopplung und Anbindung des Prothesenschaftes an der Befestigungseinrichtung und damit an dem Prothesenkniegelenk zu ermöglichen. Die Aufnahmeeinrichtung legt den Prothesenschaft drehstarr und momentenübertragend an dem Bügel fest, so dass eine Relativverlagerung des Prothesenschaftes zu dem Unterteil des Prothesenkniegelenkes und aufgrund der starren Anbindung der zumindest einen Strebe an dem Unterteil auch eine Relativverlagerung des Prothesenschaftes zu der Strebe über den Bügel zu einer Verschwenkung um die Schaftschwenkachse und aufgrund der exzentrischen Lagerung des Kraftübertragungselementes und dessen Kopplung mit dem Oberteil zu einer Relativbewegung des Oberteils des Prothesenkniegelenkes zu dem Unterteil des Prothesenkniegelenkes führt.

Der distale Lagerpunkt der Kraftübertragungseinrichtung kann an einem Träger angeordnet sein, der eine Koppeleinrichtung zur Festlegung an dem Oberteil aufweist. Die Koppeleinrichtung kann als Teil eines Pyramidenadapters ausgebildet sein, die an dem Träger ausgebildet oder angeordnet ist. Der Träger selbst kann beispielsweise als Platte oder Ausleger ausgebildet sein, der eine Aufnahme oder mehrere Aufnahmen zur Festlegung, insbesondere schwenkbaren Festlegung der Kraftübertragungseinrichtung aufweist. Durch die schwenkbare Lagerung sowohl an dem distalen Lagerpunkt als auch an dem proximalen Lagerpunkt ist es möglich, die Drehbewegung des Prothesenschaftes relativ zu der Strebe oder den Streben in eine Linearbewegung umzusetzen, die wiederum in eine Drehbewegung des Oberteils um die Prothesenkniegelenksachse umgesetzt wird. Der Kraftvektor von dem proximalen Lagerpunkt zu dem distalen Lagerpunkt ist dabei so ausgerichtet, dass er über den Verlauf der gesamten Verschwenkbewegung des Prothesenschaftes beabstandet zu der Prothesenkniegelenksachse orientiert ist, so dass keine Totpunktlage und damit eine Blockierung der Verschwenkung des Prothesenschaftes über den gewollten Verschwenkbereich hinaus auftritt.

Eine Weiterbildung der Erfindung sieht vor, dass das Kraftübertragungselement als ein Bügel mit zwei Schenkeln ausgebildet ist, die medial und lateral des Prothesenschaftes angeordnet sind. Die Bügel bilden zwei proximale Lagerpunkte aus und sind distal über einen Querträger miteinander verbunden. Der Querträger und die Schenkel sind schwenkbar um eine Schwenkachse miteinander verbunden. Die Schwenkachse der beiden Schenkel ist beabstandet zu der Prothesenkniegelenksschwenkachse. Das Kraftübertragungselement ist an dem proximalen Lagerpunkt schwenkbar gelagert. Bevorzugt ist das Kraftübertragungselement auch an dem distalen Lagerpunt schwenkbar gelagert.

Der Prothesenschaft kann eine formstabile Endkappe aufweisen, an der die proximalen Lagerpunkte oder der zumindest eine proximale Lagerpunkt ausgebildet ist. Alternativ oder ergänzend kann ein distales Befestigungselement zur Kopplung mit der Strebe an dem Prothesenschaft angeordnet sein, so dass beispielsweise eine Befestigung des Prothesenschaftes an einem Adapter oder an einem schwenkbaren Bügel möglich ist.

Die Halterung kann lösbar an dem Unterteil oder einem Unterschenkelrohr festlegbar sein. Bevorzugt ist die Halterung auswechselbar an der Strebe angeordnet, so dass eine Strebe durch Austausch der jeweiligen Halterung an unterschiedliche Prothesenkniegelenke ankoppelbar ist. Die Halterung legt die Strebe oder die Streben starr relativ zu dem Unterteil fest.

Der Prothesenschaft weist ein distales Ende auf, das bevorzugt distal über die Schaftschwenkachse hinaus ragt. Die Schaftschwenkachse ist bevorzugt im Bereich einer natürlichen Kniegelenksachse angeordnet, so dass verglichen mit dem unversorgten Bein gleiche Segmentlängen erreicht werden können. Die Erfindung betrifft ebenfalls ein Prothesenkniegelenk mit einer wie oben beschriebenen Befestigungseinrichtung.

Die Entfernung des proximalen Lagerpunktes der Kraftübertragungseinrichtung zu der Schaftschwenkachse kann veränderlich ausgebildet sein. So können beispielsweise mehrere zueinander beabstandete Formschlusselemente, wie Bohrungen, Gewinde oder Bolzen an dem Prothesenschaft oder einem Hebel angeordnet sein, um die Position der Kraftübertragungseinrichtung relativ zu der Schaftschwenkachse zu verändern. Ebenso ist es möglich, den Abstand der proximalen Lagerpunkte und der distalen Lagerpunkte voneinander veränderbar auszugestalten, beispielsweise durch eine Vielzahl zueinander beabstandeter Lagerbohrungen. Auch ist es möglich, den Abstand des distalen Lagerpunktes von der Kniegelenksschwenkachse einstellbar auszugestalten. Statt einer Veränderbarkeit der jeweiligen Abstände über unterschiedliche, in festgelegten Abstände angeordnete Befestigungselemente kann auf eine verschiebliche und fixierbare Anordnung der Lagerpunkte oder Lagerstellen ausgebildet sein, beispielsweise über Langlochführungen und klemmenden Fixierungen. Rasteinrichtungen, wie Verzahnungen, können alternativ zu einer stufenlosen Verschieblichkeit und Fixierbarkeit vorgesehen sein.

Nachfolgend wird ein Ausführungsbeispiel der Erfindung anhand der beigefügten Figuren näher erläutert. Es zeigen:
- Figur 1-: eine schematische Frontalansicht eines Prothesenkniegelenkes mit einer Befestigungseinrichtung;
- Figur 2 -: eine Seitenansicht;
- Figur 3 -: eine Seitenansicht einer Variante in gestreckter Position
- Figur 4 -: die Variante gemäß Figur 3 in flektierter Position; sowie
- Figur 5: die Variante gemäß Figur 3 in Rückansicht.

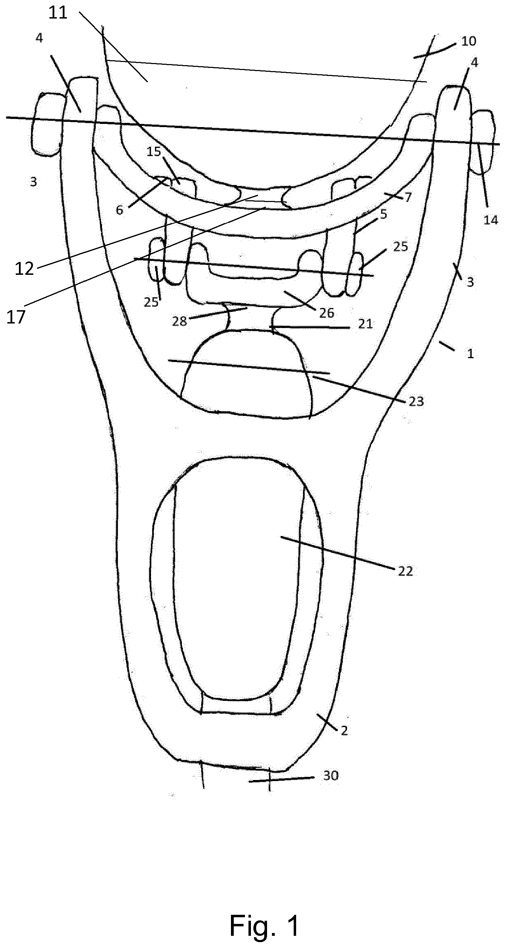

Figur 1 zeigt in einer schematischen frontalen Darstellung ein Prothesenkniegelenk 20 mit einem Oberteil 21 und einem Unterteil 22, die um eine Schwenkachse 23 schwenkbar miteinander verbunden sind. Zwischen dem Oberteil 21 und dem Unterteil 22 können Dämpfereinrichtungen, Aktuatoren, Endanschläge sowie Steuereinrichtungen und Antriebe zum Verstellen von Widerständen, Anschlägen oder dergleichen angeordnet sein. An dem Unterteil 22 ist distal ein Unterschenkelrohr 30 angeordnet, das das Prothesenkniegelenk 20 mit einem nicht dargestellten Prothesenfuß verbindet. An dem Oberteil 21 können übliche Anschlussmittel zur Festlegung an einem Prothesenschaft angeordnet sein, beispielsweise ein Pyramidenadapter.

An dem Prothesenkniegelenk 20 ist eine Befestigungseinrichtung 1 angeordnet, die in dem distalen Bereich eine Halterung 2 aufweist, um die Befestigungseinrichtung 1 an dem Unterteil 22 oder, wie in dem dargestellten Ausführungsbeispiel gezeigt, an dem Unterschenkelrohr 30 festzulegen. Über die Halterung 2 wird die Befestigungseinrichtung 1 starr mit dem Unterteil 22 oder dem Unterschenkelrohr 30 gekoppelt, beispielsweise über Formschlusselemente oder über Klemmeinrichtungen.

Die Befestigungseinrichtung 1 weist zwei sich von der distalen Halterung 2 in Proximalrichtung erstreckende Streben 3 oder Schenkel 3 auf, die medial und lateral des Prothesenkniegelenkes 20 angeordnet sind. Die beiden Streben 3 oder Schenkel 3 sind miteinander verbunden, so dass sich eine U-förmige Ausgestaltung im proximalen Bereich der Befestigungseinrichtung 1 ausbildet. An den proximalen Enden der Streben 3 oder Schenkel 3 sind Aufnahmen 4 ausgebildet, beispielsweise Lagerzapfen, Lagerbohrungen oder ähnliches, durch die eine Schaftschwenkachse 14 verläuft. Statt zweier Streben 3 oder Schenkel 3 kann in einer Variante der Erfindung auch nur eine Strebe 3 oder ein Schenkel 3 ausgebildet sein, entweder medial oder lateral dem Prothesenkniegelenk 2. Eine Ausgestaltung mit zwei Streben 3 oder Schenkeln 3 erhöht die Stabilität und die Führungsgenauigkeit.

Um die Schaftschwenkachse 14 ist ein Bügel 7 schwenkbar gelagert, der ebenfalls U-förmig ausgebildet ist und seinen tiefsten Punkt, also seinen maximal distalen Punkt, in der Mitte zwischen den beiden Streben 3 oder Schenkeln 3 aufweist. An dem Bügel 7 ist eine Aufnahmeeinrichtung 17 angeordnet, beispielsweise ein Pyramidenadapter, der zur Festlegung eines Prothesenschaftes 10 dient. An dem Prothesenschaft 10, der als formstabiler Prothesenschaft zur Aufnahme eines Oberschenkelstumpfes oder mit einer formstabilen Endkappe 11 ausgebildet ist, insbesondere zur Aufnahme eines Oberschenkelstumpfes nach einer Knieexartikulation, ist ein distales Befestigungselement 12 angeordnet, beispielsweise eine Pyramidenadapteraufnahme, um eine reversible, formschlüssige und ausrichtbare Befestigung des Prothesenschaftes 10 an der Aufnahmeeinrichtung 17 vornehmen zu können. Der Prothesenschaft 10 ist über das distale Befestigngselement 12 und die Aufnahmeeinrichtung 17 drehfest und momentenübertragend an dem Bügel 7 fixiert. Über die Festlegung an dem Bügel 7 kann der Prothesenschaft 10 um die Schaftschwenkachse 14 verschwenkt werden. Die Schaftschwenkachse 14 verläuft proximal zu dem distalen Ende des Prothesenschaftes 10. Durch die reversible Festlegung über das distale Befestigungslement 12 an der Aufnahmeeinrichtung 17 kann ein einfacher Austausch der Komponenten erfolgen.

An dem Bügel 7 selbst ist ein Ausleger oder Hebel 6 befestigt, der starr mit dem Bügel 7 gekoppelt ist. Der Hebel 6 erstreckt sich in rückwärtiger Richtung, also in posteriore Richtung, und bildet jeweils einen proximalen Lagerpunkt 15 aus. Es sind zwei Hebel 6 an dem Bügel 7 ausgebildet oder befestigt und symmetrisch zur Längserstreckung des Prothesenkniegelenkes 20 angeordnet. Von dem proximalen Lagerpunkt 15 an dem Hebel 6 erstreckt sich jeweils ein Kraftübertragungselement 5 in distale Richtung, wo das jeweilige Kraftübertragungselement 5 an einem distalen Lagerpunkt 25 an einem Träger 26 gelagert ist. Das Kraftübertragungselement 5 ist sowohl an dem proximalen Lagerpunkt 15 als auch an dem distalen Lagerpunkt 20 schwenkbar gelagert. Der Träger 26 verbindet die beiden Kraftübertragungselemente 5 miteinander, so dass ein weiterer, U-förmiger Bügel gebildet wird. Der Träger 26 weist eine Koppeleinrichtung 28 auf, die mit den oberen Anschlussmitteln des Oberteils 21 korrespondiert, also beispielsweise eine Pyramidenadapteraufnahme ausbildet, ähnlich der Aufnahmeeinrichtung an dem Prothesenschaft 10. Verschwenkt der Prothesenschaft 10 um die Schaftschwenkachse 14 und flektiert den Prothesenschaft 10 und damit den Stumpf relativ zu dem Unterschenkelrohr 30 oder dem Unterteil 22, verschwenkt der Bügel 7 und damit die beiden Hebel 6, so dass die Kraftübertragungseinrichtungen 5 in distale Richtung verlagert werden. Aufgrund der distalen Verlagerung der Kraftübertragungseinrichtungen 5 werden die distalen Lagerpunkte 25 mit einer Druckkraft beaufschlagt, Aufgrund der exzentrischen Anordnung der Lagerpunkte 25 relativ zu der Kniegelenkschwenkachse 23 entsteht ein Moment um die Prothesenkniegelenksachse 23, so dass das Oberteil 21 um die Prothesenkniegelenksachse 23 relativ zu dem Unterteil 22 verschwenkt wird.

Die Schaftschwenkachse 14 bildet somit für den Prothesennutzer diejenige Achse aus, um die die gesamte prothetische Einrichtung, abgesehen von dem Prothesenschaft 10, verschwenkt. Durch die Positionierung der Schaftschwenkachse 14 in dem Bereich des distalen Endes des Prothesenschaftes 10, jedoch proximal zu dem distalen Ende des Prothesenschaftes 10 ist es möglich, die für den Nutzer relevante Schwenkachse nahe der natürlichen Kniegelenksachse zu positionieren. Dadurch ist es möglich, nahezu identische Segmentlängen von Oberschenkel und Unterschenkel bzw. Oberschenkelstumpf und distale Prothesenkomponente bereitzustellen. Eine konstruktive Veränderung herkömmlicher Prothesenkniegelenke 20 ist nicht weiter notwendig, sowohl die Anschlusseinrichtungen als auch die Abstimmungen und die Programmierungen können unverändert beibehalten werden. Unterschiedliche Beinlängen können einfach durch eine Verlagerung der Position der Schaftschwenkachse 14 ausgeglichen werden, ebenso können Übersetzungsverhältnisse durch eine Einstellung und Modifikation der Position der jeweiligen Lagerpunkte 15, 25 bzw. der Befestigung des Prothesenschaftes 10 an den Streben 3 oder Schenkeln 3 oder der Strebe 3 oder dem Schenkel 3 verändert werden.

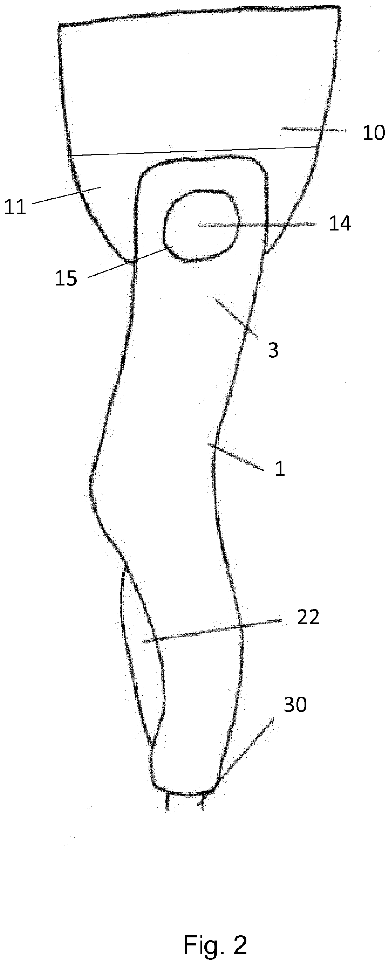

Figur 2 zeigt in einer Seitenansicht die Befestigungseinrichtung 1 mit der Schaftschwenkachse 24 an dem proximalen Ende der Streben 3 oder Schenkel 3, dem Unterschenkelrohr 30 und dem Prothesenkniegelenkunterteil 22. Der Prothesenschaft 10 erstreckt sich in distaler Richtung über die Schaftschwenkachse 14 hinaus. An dem Prothesenschaft 10 ist eine formstabile Endkappe 11 angeordnet oder ausgebildet, über die eine Festlegung des Prothesenschaftes 10 an den Streben 3 oder Schenkeln 3 erfolgen kann. Die Festlegung kann über proxmale Lagerpunkte 15 erfolgen, so dass die Endkappe 11 direkt oder über Zwischenstücke an den Streben 3 oder Schenkeln 3 schwenkbar festgelegt werden können.

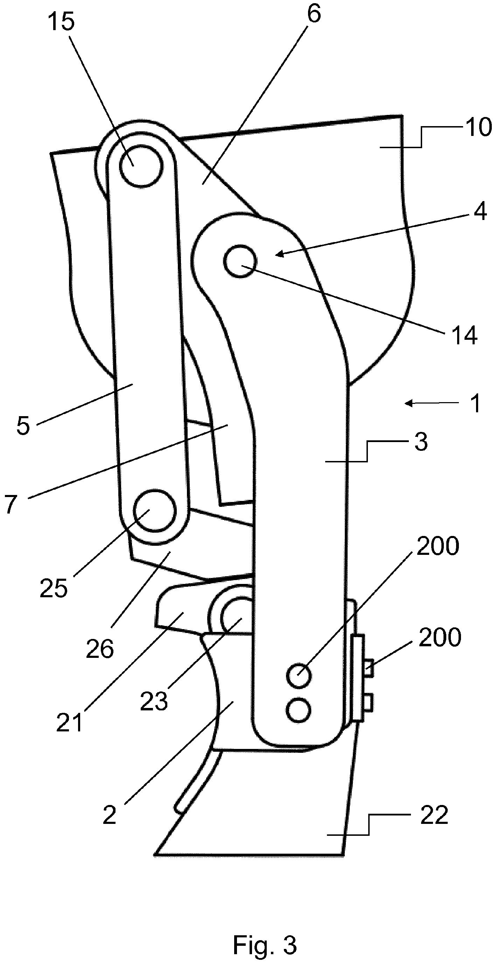

Figur 3 zeigt in einer gestreckten Stellung eine Variante der Erfindung in einer schematischen Schnittansicht. Die Befestigungseinrichtung 1, die als Adapter zur Festlegung des Prothesenschaftes 10 an einem herkömmlichen Prothesenkniegelenk 20 ausgebildet ist, ist mit ihrem distalen Ende über eine Halterung 2, die auswechselbar an der Befestigungseinrichtung 1 angeordnet sein kann, lösbar direkt mit dem Unterteil 22 des Prothesenkniegelenkes 20 oder in einer Variante mit dem Unterschenkelrohr 30 verbunden. Die Streben 3 oder Schenkel 3 erstrecken sich in proximaler Richtung bis über das distale Ende des Prothesenschaftes 10 hinaus. Die Schaftschwenkachse 14 liegt proximal zu dem distalen Schaftende des Prothesenschaftes 10, und die Schenkel des Bügels 7 erstrecken sich distal zu der nicht zu erkennenden Aufnahmeeinrichtung 17, die zur Aufnahme des Prothesenschaftes 10 dient. In der Seitenansicht ist zu erkennen, dass die Hebel 6 in posteriorer, also in die in Gehrichtung hinterer Richtung von der Anbindungsstelle an der Aufnahmeeinrichtung 17 überstehen, so dass ein Hebelarm ausgebildet wird, so dass bei einer Verschwenkung des Prothesenschaftes 10 um die Schaftschwenkachse 14 das Kraftübertragungselement 5, das schwenkbar an dem proximalen Lagerpunkt 15 und dem distalen Lagerpunkt 25 gelagert ist, im Wesentlichen geradlinig nach unten bewegt wird.

Der distale Lagerpunkt 25 ist an dem Träger 26 ebenfalls in Gehrichtung posterior, also hinter der Kniegelenksschwenkachse 23 angeordnet, so dass der Kraftvektor von dem proximalen Lagerpunkt 15 zum distalen Lagerpunkt 25 nicht die Kniegelenksschwenkachse 23 schneidet. Dadurch wird ein Moment um die Prothesenkniegelenksschwenkachse 23 erzeugt, so dass das Oberteil 21 um die Prothesenkniegelenksschwenkachse 23 relativ zu dem Unterteil 22 verschwenkt wird.

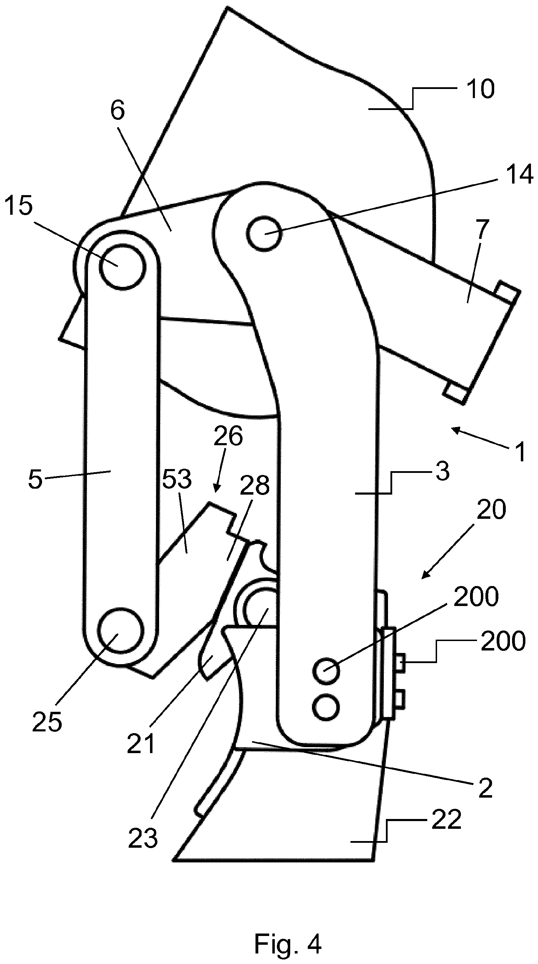

Die verschwenkte Stellung ist in der Figur 4 gezeigt.

Die Befestigungseinrichtung 1 ermöglicht die Versetzung des effektiven Kniedrehpunktes auf die anatomische Höhe, indem die Schaftschwenkachse 14 eingeführt wird. Konventionelle Prothesenkniegelenke 20 können auch für Patienten mit langen Oberschenkelstümpfen, insbesondere Knieexartikulationspatienten, angewendet werden. Eine Momentenübertragung findet von der Befestigungseinrichtung 1 auf die herkömmlichen Prothesenkniegelenke statt. Die Kopplung des Prothesenschaftes 10 mit der Befestigungseinrichtung 1 erfolgt entweder über einen herkömmlichen Pyramidenadapter oder aber über einlaminierte Anbindungspunkte, über die die Schenkel medial und lateral mit dem Prothesenschaft 10 verbunden werden können, gegebenenfalls unter Einsatz von Distanzstücken.

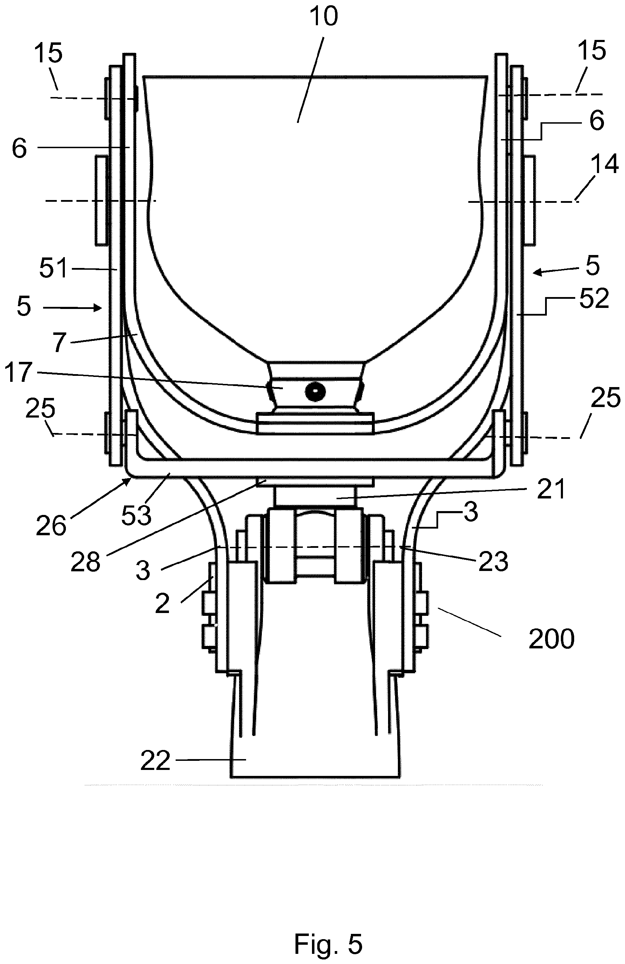

Figur 5 zeigt die Variante gemäß der Figuren 3 und 4 in einer rückwärtigen Ansicht. In der Figur 5 ist zu erkennen, dass der Prothesenschaft 10 an seinem distalen Ende über einen Pyramidenadapter als Aufnahmeeinrichtung 17 an dem Bügel 7 zentral festgelegt ist. Die Ausgestaltung der Aufnahmeeinrichtung 17 als Pyramidenadapter hat den Vorteil, dass herkömmliche Prothesenschäfte 10 verwendet werden können, die bereits zur unmittelbaren Befestigung an einem Prothesenkniegelenk ausgebildet sind. Alternativ kann der Prothesenschaft 10 mit anderen Befestigungseinrichtungen an dem Bügel 7 oder über andere Einrichtungen mit dem oder den Kraftübertragungselementen 5 gekoppelt sein. In dem dargestellten Ausführungsbeispiel ist der Bügel 7 über die Schaftschwenkachse 14 in Proximalrichtung hinausgehend verlängert, sodass die Hebel 6 eine Verbindung zwischen der Schaftschwenkachse 14 und den Lagerpunkten 15 ausbilden Dadurch wird eine Kraftkopplung zwischen dem Prothesenschaft 10 und den proximalen Lagerpunkten 15 bereitgestellt, sodass bei einer Verschwenkung des Prothesenschaftes 10 um die Schaftschwenkachse 14 die Schenkel 51, 52 des Kraftübertragungselementes 5 aufgrund der schwenkbaren Lagerung in den Lagerpunkten eine Kraft in Distalrichtung auf die distalen Lagerpunkte ausüben. Dadurch wird der Träger 26, der als Querträger 53 den medialen und lateralen Schenkel 51, 52 miteinander verbindet, um die Prothesenkniegelenksachse 23 verschwenkt.

Die distale Halterung ist über Bolzen oder Schrauben 200 lösbar an dem Unterteil 22 befestigt. Eine Dämpfereinrichtung, wie sie in der Figur 1 dargestellt ist, ist in der Variante gemäß der Figuren 3 bis 5 nicht dargestellt. Eine solche Dämpfereinrichtung kann an dem Oberteil 21 und dem Unterteil 22 befestigt werden. Die Streben 3 sind medial und lateral an dem Unterteil 22 über die Schrauben oder Bolzen 200 festgelegt, statt einer formschlüssigen Befestigung der Streben 3 kann auch eine Klemmung an dem Unterteil 22 erfolgen.

## Patentansprüche

1. Befestigungseinrichtung (1) zur Befestigung eines Prothesenschaftes (10) an einem Prothesenkniegelenk (20), das ein proximales Oberteil (21) und ein distales Unterteil (22) aufweist, die schwenkbar um eine Prothesenkniegelenksachse (23) aneinander gelagert sind, wobei
die Befestigungseinrichtung (1) eine distale Halterung (2) zur Festlegung an dem Unterteil (22) oder einem Unterschenkelrohr (30) aufweist, **dadurch gekennzeichnet, dass**
- sich von der Halterung (2) zumindest eine Strebe (3) in Proximalrichtung über die Prothesenkniegelenksachse (23) hinaus erstreckt,
- an der Strebe (3) proximal zu der Prothesenkniegelenksachse (23) eine Aufnahme (4) zur schwenkbaren Lagerung des Prothesenschaftes (10) um eine Schaftschwenkachse (14) angeordnet ist,
- versetzt zu der Schaftschwenkachse (14) zumindest ein Kraftübertragungselement (5) an einem proximalen Lagerpunkt (15) befestigt ist, um eine Schwenkbewegung des Prothesenschaftes (10) zu übertragen,
- das Kraftübertragungselement (5) an einem distalen Lagerpunkt (25) mit dem Oberteil (21) dergestalt gekoppelt ist, dass eine Schwenkbewegung des Prothesenschaftes (10) um die Schaftschwenkachse (14) eine Schwenkbewegung des Oberteiles (21) um die Prothesenkniegelenksachse (23) bewirkt.

2. Befestigungseinrichtung nach Anspruch 1, **dadurch gekennzeichnet, dass** der proximale Lagerpunkt (15) an dem Prothesenschaft (10) oder an einem Hebel (6) angeordnet ist, der um die Schaftschwenkachse (14) schwenkbar und mit dem Prothesenschaft (10) gekoppelt ist.

3. Befestigungseinrichtung nach Anspruch 1 oder 2, **dadurch gekennzeichnet, dass** an der zumindest einen Strebe (3) ein Bügel (7) mit einer Aufnahmeeinrichtung (17) zur Festlegung des Prothesenschaftes (10) an dem Bügel (7) gelagert ist.

4. Befestigungseinrichtung nach Anspruch 3, **dadurch gekennzeichnet, dass** die Aufnahmeeinrichtung (17) als Teil eines Pyramidenadapters ausgebildet ist.

5. Befestigungseinrichtung nach Anspruch 3 oder 4, **dadurch gekennzeichnet, dass** der Bügel (7) als einarmiger oder zweiarmiger Bügel ausgebildet ist.

6. Befestigungseinrichtung nach einem der voranstehenden Ansprüche, **dadurch gekennzeichnet, dass** der distale Lagerpunkt (25) an einem Träger (26) angeordnet ist, der eine Koppeleinrichtung (28) zur Festlegung an dem Oberteil (21) aufweist.

7. Befestigungseinrichtung nach Anspruch 6, **dadurch gekennzeichnet, dass** die Koppeleinrichtung (28) als Teil eines Pyramidenadapters ausgebildet ist.

8. Befestigungseinrichtung nach einem der voranstehenden Ansprüche, **dadurch gekennzeichnet, dass** das Kraftübertragungselement (5) als ein Bügel mit zwei Schenkeln (51, 52) ausgebildet ist, die medial und lateral des Prothesenschaftes (10) angeordnet sind, zwei proximale Lagerpunkte (15) ausbilden und distal über einen Querträger (53) miteinander verbunden sind.

9. Befestigungseinrichtung nach einem der voranstehenden Ansprüche, **dadurch gekennzeichnet, dass** das Kraftübertragungselement (5) schwenkbar um den proximalen Lagerpunkt (15) gelagert ist.

10. Befestigungseinrichtung nach einem der voranstehenden Ansprüche, **dadurch gekennzeichnet, dass** der Prothesenschaft (10) eine formstabile Endkappe (11) aufweist, an der der zumindest ein proximaler Lagerpunkt (15) ausgebildet ist und/oder ein distales Befestigungselement (12) zur Kopplung mit der Strebe (3) angeordnet ist.

11. Befestigungseinrichtung nach einem der voranstehenden Ansprüche, **dadurch gekennzeichnet, dass** die Halterung (2) lösbar an dem Unterteil (22) oder einem Unterschenkelrohr (30) festlegbar ist.

12. Befestigungseinrichtung nach einem der voranstehenden Ansprüche, **dadurch gekennzeichnet, dass** der Prothesenschaft (10) ein distales Ende aufweist, das distal über die Schaftschwenkachse (14) hinausragt.

13. Befestigungseinrichtung nach einem der voranstehenden Ansprüche, **dadurch gekennzeichnet, dass** die Schaftschwenkachse (14) im Bereich einer natürlichen Kniegelenksachse angeordnet ist.

14. Prothesenkniegelenk (20) mit einer Befestigungseinrichtung (1) nach einem der voranstehenden Ansprüche.

## Claims

1. Fastening device (1) for fastening a prosthesis socket (10) to a prosthetic knee joint (20) that has a proximal top part (21) and a distal bottom part (22), which are mounted on each other pivotably about a prosthetic knee joint axis (23), wherein the fastening device (1) has a distal holder (2) for securing to the bottom part (22) or to a lower-leg tube (30), **characterized in that**
- at least one strut (3) extends in a proximal direction from the holder (2) beyond the prosthetic knee joint axis (23),
- a receptacle (4) is arranged on the strut (3), proximally with respect to the prosthetic knee joint axis (23), for the pivotable mounting of the prosthesis socket (10) about a socket pivot axis (14),
- at least one force transmission element (5), offset with respect to the socket pivot axis (14), is fastened at a proximal bearing point (15) in order to transmit a pivoting movement of the prosthesis socket (10),
- the force transmission element (5) is coupled to the top part (21), at a distal bearing point (25), in such a way that a pivoting movement of the prosthesis socket (10) about the socket pivot axis (14) causes a pivoting movement of the top part (21) about the prosthetic knee joint axis (23).

2. Fastening device according to Claim 1, **characterized in that** the proximal bearing point (15) is arranged on the prosthesis socket (10), or on a lever (6) which is pivotable about the socket pivot axis (14) and coupled to the prosthesis socket (10).

3. Fastening device according to Claim 1 or 2, **characterized in that** a bracket (7), with a receiving device (17) for securing the prosthesis socket (10) to the bracket (7), is mounted on the at least one strut (3).

4. Fastening device according to Claim 3, **characterized in that** the receiving device (17) is designed as part of a pyramid adapter.

5. Fastening device according to Claim 3 or 4, **characterized in that** the bracket (7) is designed as a one-arm or two-arm bracket.

6. Fastening device according to one of the preceding claims, **characterized in that** the distal bearing point (25) is arranged on a carrier (26) which has a coupling device (28) for securing to the top part (21).

7. Fastening device according to Claim 6, **characterized in that** the coupling device (28) is designed as part of a pyramid adapter.

8. Fastening device according to one of the preceding claims, **characterized in that** the force transmission element (5) is designed as a bracket with two branches (51, 52) which are arranged medially and laterally with respect to the prosthesis socket (10), form two proximal bearing points (15), and are connected to each other distally via a crosspiece (53).

9. Fastening device according to one of the preceding claims, **characterized in that** the force transmission element (5) is mounted pivotably about the proximal bearing point (15).

10. Fastening device according to one of the preceding claims, **characterized in that** the prosthesis socket (10) has a dimensionally stable end cap (11) on which at least one proximal bearing point (15) is formed and/or a distal fastening element (12) is arranged for coupling to the strut (3).

11. Fastening device according to one of the preceding claims, **characterized in that** the holder (2) can be secured releasably to the bottom part (22) or to a lower-leg tube (30).

12. Fastening device according to one of the preceding claims, **characterized in that** the prosthesis socket (10) has a distal end which protrudes distally beyond the socket pivot axis (14).

13. Fastening device according to one of the preceding claims, **characterized in that** the socket pivot axis (14) is arranged in the region of a natural knee joint axis.

14. Prosthetic knee joint (20) having a fastening device (1) according to one of the preceding claims.

## Revendications

1. Dispositif de fixation (1) pour fixer une emboîture de prothèse (10) à une articulation de genou prothétique (20) comprenant une partie supérieure proximale (21) et une partie inférieure distale (22) qui sont montées l'une sur l'autre de manière à pouvoir pivoter autour d'un axe d'articulation de genou prothétique (23),
le dispositif de fixation (1) comportant un élément de maintien distal (2) pour l'immobilisation à la partie inférieure (22) ou à un tube de bas de jambe (30),
**caractérisé en ce que**
- au moins une entretoise (3) s'étend depuis l'élément de maintien (2) dans la direction proximale au-delà de l'axe d'articulation de genou prothétique (23),
- un logement (4) pour le montage pivotant de l'emboîture de prothèse (10) autour d'un axe de pivotement d'emboîture (14) est disposé sur l'entretoise (3) de manière proximale par rapport à l'axe d'articulation de genou prothétique (23),
- au moins un élément de transmission de force (5) est fixé à un point d'appui proximal (15) en étant décalé de l'axe de pivotement d'emboîture (14), afin de transmettre un mouvement de pivotement de l'emboîture de prothèse (10),
- l'élément de transmission de force (5) est couplé à la partie supérieure (21) au niveau d'un point d'appui distal (25), de telle sorte qu'un mouvement de pivotement de l'emboîture de prothèse (10) autour de l'axe de pivotement d'emboîture (14) provoque un mouvement de pivotement de la partie supérieure (21) autour de l'axe d'articulation de genou prothétique (23).

2. Dispositif de fixation selon la revendication 1, **caractérisé en ce que** le point d'appui proximal (15) est disposé sur l'emboîture de prothèse (10) ou sur un levier (6) qui peut pivoter autour de l'axe de pivotement d'emboîture (14) et qui est couplé à l'emboîture de prothèse (10).

3. Dispositif de fixation selon la revendication 1 ou 2, **caractérisé en ce qu'**un étrier (7) comprenant un dispositif de réception (17) pour immobiliser l'emboîture de prothèse (10) à l'étrier (7) est monté sur ladite au moins une entretoise (3).

4. Dispositif de fixation selon la revendication 3, **caractérisé en ce que** le dispositif de réception (17) est réalisé sous forme de partie d'un adaptateur pyramidal.

5. Dispositif de fixation selon la revendication 3 ou 4, **caractérisé en ce que** l'étrier (7) est réalisé sous forme d'étrier à un bras ou à deux bras.

6. Dispositif de fixation selon l'une des revendications précédentes, **caractérisé en ce que** le point d'appui distal (25) est disposé sur un support (26) qui présente un dispositif d'accouplement (28) pour l'immobilisation à la partie supérieure (21).

7. Dispositif de fixation selon la revendication 6, **caractérisé en ce que** le dispositif d'accouplement (28) est réalisé sous forme de partie d'un adaptateur pyramidal.

8. Dispositif de fixation selon l'une des revendications précédentes, **caractérisé en ce que** l'élément de transmission de force (5) est réalisé sous forme d'étrier comportant deux branches (51, 52) qui sont disposées médialement et latéralement par rapport à l'emboîture de prothèse (10), qui forment deux points d'appui proximaux (15) et qui sont reliées entre elles distalement par une traverse (53).

9. Dispositif de fixation selon l'une des revendications précédentes, **caractérisé en ce que** l'élément de transmission de force (5) est monté pivotant autour du point d'appui proximal (15).

10. Dispositif de fixation selon l'une des revendications précédentes, **caractérisé en ce que** l'emboîture de prothèse (10) présente un capuchon d'extrémité (11) solide en forme, sur lequel est réalisé ledit au moins un point d'appui proximal (15) et/ou sur lequel est disposé un élément de fixation distal (12) pour l'accouplement avec l'entretoise (3).

11. Dispositif de fixation selon l'une des revendications précédentes, **caractérisé en ce que** l'élément de maintien (2) peut être immobilisé de manière amovible à la partie inférieure (22) ou à un tube de bas de jambe (30).

12. Dispositif de fixation selon l'une des revendications précédentes, **caractérisé en ce que** l'emboîture de prothèse (10) présente une extrémité distale qui dépasse distalement au-delà de l'axe de pivotement d'emboîture (14).

13. Dispositif de fixation selon l'une des revendications précédentes, **caractérisé en ce que** l'axe de pivotement d'emboîture (14) est disposé au niveau d'un axe d'articulation de genou naturel.

14. Articulation de genou prothétique (20) comportant un dispositif de fixation (1) selon l'une des revendications précédentes.
